# EUROPEAN PATENT APPLICATION

(11) **EP 3 309 794 A1**
(43) Date of publication of application: **18.04.2018**
(21) Application number: 17196192.3
(22) Date of filing: 12.10.2017
(51) Int. Cl.: G16H 40/63, G16H 40/67

(54) **MEDICAL CARE APPARATUS COMPRISING A SECOND DISPLAY FOR DISPLAYING OPERATIONAL PARAMETERS**

(30) Priority: 13.10.2016 JP 2016201555
(71) Applicant: J. MORITA MANUFACTURING CORPORATION, Fushimi-ku, Kyoto-shi, Kyoto 6128533 (JP)
(72) Inventor: TSURUTA, Yuki, Kyoto, 612-8533 (JP); SONOBE, Kouichi, Kyoto, 612-8533 (JP)
(74) Representative: Müller Hoffmann & Partner

(57) **Abstract**

A medical care apparatus (10) for improving the convenience at the time when a user operates an operation image is provided. The medical care apparatus (10) includes: an operation monitor (9) configured to display an operation image for operating a medical care instrument (1, 4, 2, 7); a display monitor (6) located differently from the operation monitor (9), the display monitor (6) being greater in display area than the operation monitor (9); and a main control unit (12) configured to cause the display monitor (6) to display an associated image that is different from the operation image displayed on the operation monitor (9) and that is associated with the operation image, thereby improving the convenience at the time when the user operates the operation image.

## Description

### BACKGROUND OF THE INVENTION

### Field of the Invention

The present invention relates to a medical care apparatus.

### Description of the Background Art

Conventionally, a medical care apparatus for conducting medical care in the healthcare field such as a dental field and a medical field includes a display unit for displaying an operation image used for operating a medical device.

For example, a medical care apparatus disclosed in Japan Patent No. 5570801 includes a display device for displaying operation details about medical devices as operation images.

### SUMMARY OF THE INVENTION

In the medical care apparatus disclosed in Japan Patent No. 5570801, an operation image is merely displayed on a display area on one screen. Accordingly, from the image displayed on this limited display area, it is difficult for a user to obtain sufficient information required when the user operates the operation image, thereby leading to lack of convenience.

The present invention has been made to solve the above-described problems. An object of the present invention is to provide a medical care apparatus that allows an improvement in convenience at the time when a user operates an operation image.

A medical care apparatus according to the present invention includes: a first display unit configured to display an operation image for operating a medical care instrument; a second display unit located differently from the first display unit, the second display unit being greater in display area than the first display unit; and a display control unit configured to cause the second display unit to display an associated image that is different from the operation image displayed on the first display unit, the associated image being associated with the operation image.

Preferably, the associated image includes detailed information associated with the operation image displayed on the first display unit.

Preferably, the display control unit is configured to switch the associated image displayed on the second display unit according to an operation performed by a user on the operation image displayed on the first display unit.

Preferably, the display control unit is configured to switch the associated image displayed on the second display unit in accordance with a type of a selected medical care instrument.

Preferably, the associated image includes information required when a user operates the operation image.

Preferably, the operation image is operated by a user when the user sets a function of the medical care instrument, and the associated image includes information required when the user sets the function of the medical care instrument.

Preferably, the operation image is operated by a user when the user performs maintenance of the medical care instrument, and the associated image includes information required when the user performs maintenance of the medical care instrument.

Preferably, the display control unit is configured to switch the associated image displayed on the second display unit in each progress of maintenance of the medical care instrument performed by a user.

Preferably, the operation image is operated by a user when an abnormality occurs, and the associated image includes information for notifying the user about occurrence of the abnormality.

Preferably, the operation image is operated by a user when the user obtains information from outside, and the associated image includes information obtained from outside.

According to the medical care apparatus of the present invention, the second display unit that is greater in display area than the first display unit displaying an operation image is configured to display an associated image that is different from the operation image but associated with the operation image. Accordingly, the convenience for the user operating the operation image can be improved.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a schematic diagram showing the configuration of an external appearance of a medical care apparatus.
Fig. 2 is a block diagram schematically showing the configuration of the inside of the medical care apparatus.
Fig. 3 is a diagram for illustrating display examples of an operation monitor and a display monitor in a non-associated mode.
Fig. 4 is a diagram for illustrating display examples of the operation monitor and the display monitor in an associated mode.
Fig. 5 is a diagram for illustrating display examples of the operation monitor and the display monitor in the associated mode.
Fig. 6 is a diagram for illustrating display examples of the operation monitor and the display monitor in the associated mode.
Fig. 7 is a diagram for illustrating display examples of the operation monitor and the display monitor in the associated mode.
Fig. 8 is a diagram for illustrating display examples of the operation monitor and the display monitor in the associated mode.
Fig. 9 is a diagram for illustrating display examples of the operation monitor and the display monitor in a maintenance mode.
Fig. 10 is a diagram for illustrating display examples of the operation monitor and the display monitor in the maintenance mode.
Fig. 11 is a diagram for illustrating display examples of the operation monitor and the display monitor in the maintenance mode.
Fig. 12 is a diagram for illustrating display examples of the operation monitor and the display monitor in the maintenance mode.
Fig. 13 is a diagram for illustrating display examples of the operation monitor and the display monitor in the maintenance mode.
Fig. 14 is a flowchart for illustrating a display control process performed by the medical care apparatus.

### DESCRIPTION OF THE PREFERRED EMBODIMENTS

Embodiments according to the present invention will be hereinafter described with reference to the accompanying drawings. In the present embodiment, a medical care apparatus that can be used for a dental care will be explained as one illustrative form of a medical care apparatus. In addition, the medical care apparatus according to the present embodiment is applicable not only to a dental care but also to any type of medical cares in the fields of ophthalmology, otolaryngology, radiology, veterinary science, and the like. Also, the medical care includes a diagnosis and a treatment.

### [Basic Configuration of Medical Care Apparatus]

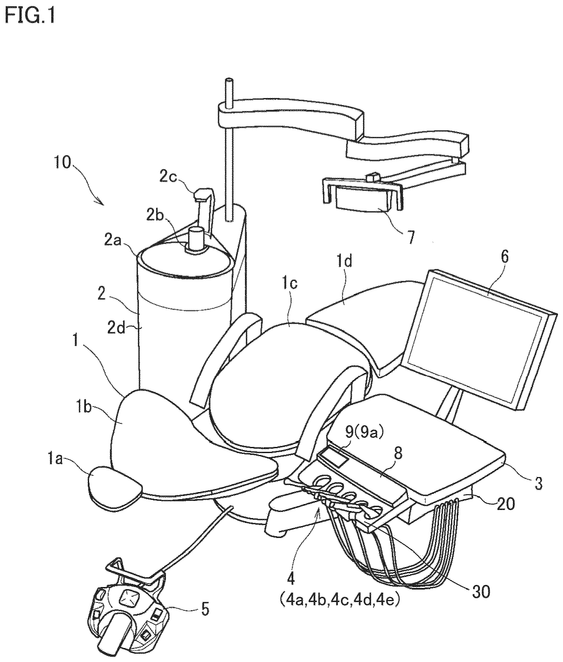

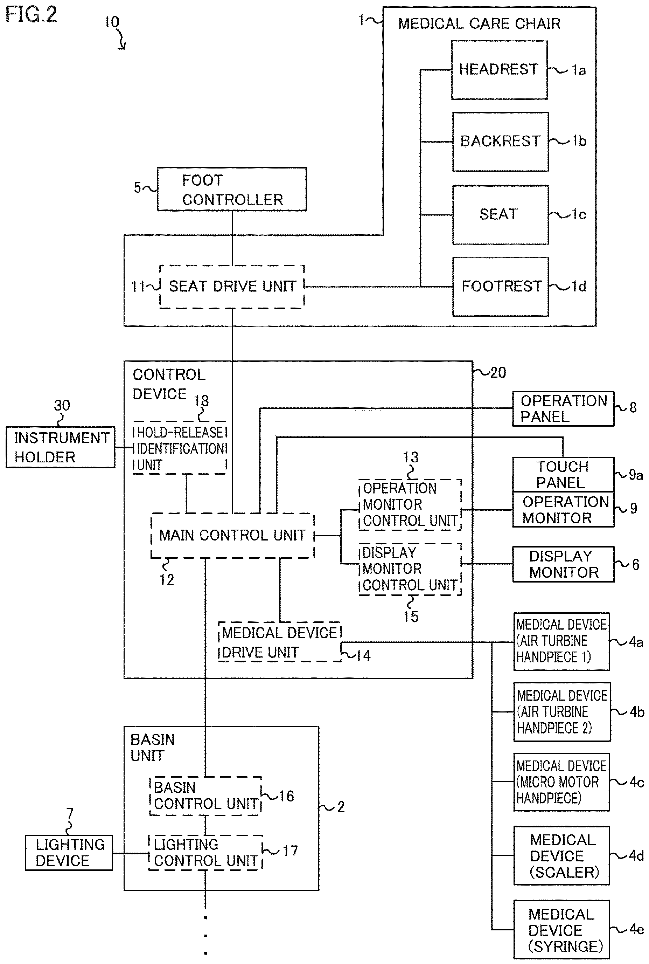

Fig. 1 is a schematic diagram showing the configuration of an external appearance of a medical care apparatus 10, and Fig. 2 is a block diagram schematically showing the configuration of the inside of medical care apparatus 10.

As shown in Figs. 1 and 2, medical care apparatus 10 (hereinafter also referred to as care apparatus or medical apparatus) includes: a medical care chair 1, a foot controller 5, a tray table 3, a control device 20, an instrument holder 30, an operation panel 8, a display monitor 6, a basin unit 2, and a lighting device 7.

Medical care chair 1 includes a headrest 1a supporting the head of a patient, a backrest 1b supporting the back of the patient, a seat 1c supporting buttocks of the patient, a footrest 1d supporting legs of the patient, and a seat drive unit 11. Seat drive unit 11 is configured by a central processing unit (CPU), a read only memory (ROM), a random access memory (RAM) and the like that are mounted on a substrate (not shown), and configured to control driving of each of headrest 1a, backrest 1b, seat 1c, and footrest 1d.

For example, seat 1c is raised or lowered under the control of seat drive unit 11. Each of headrest 1a, backrest 1b and footrest 1d is moved under the control of seat drive unit 11 in the direction that is perpendicular or horizontal to seat 1c. When headrest 1a, backrest 1b and footrest 1d are moved in the direction perpendicular to seat 1c, the patient sitting on medical care chair 1 is brought into a seated posture. When headrest 1a, backrest 1b and footrest 1d are moved in the direction horizontal to seat 1c, the patient sitting on medical care chair 1 is laid on his/her back. In this way, seat drive unit 11 has a function of driving headrest 1a, backrest 1b, seat 1c, and footrest 1d to thereby change the posture of medical care chair 1.

Foot controller 5 has a plurality of switches (pedals) configured to receive the pressing-down operation by the user's foot. The user can allocate a prescribed function to each of the plurality of switches.

For example, the user can allocate the function of changing the posture of medical care chair 1 to a switch on foot controller 5. When the user uses his/her foot to press down the switch to which the function of changing the posture of medical care chair 1 is allocated, foot controller 5 outputs a control signal to seat drive unit 11. Based on the control signal, seat drive unit 11 drives headrest 1a, backrest 1b, seat 1c, and footrest 1d.

Furthermore, the user can allocate the function of driving medical device 4 described later to a switch on foot controller 5. When the user uses his/her foot to press down the switch to which the function of driving medical device 4 is allocated, foot controller 5 outputs a control signal to medical device drive unit 14 described later. Based on the control signal, medical device drive unit 14 controls driving of medical device 4.

Other functions such as functions of controlling a lighting device 7 to be turned on and off can be allocated to switches on foot controller 5.

Tray table 3 is used as a table on which tools and the like are placed during the medical care. Tray table 3 is connected to an arm (not shown) extending from medical care chair 1 or a floor. Accordingly, the user can manually move tray table 3 pivotally, horizontally and perpendicularly relative to medical care chair 1.

Control device 20 is provided on the bottom surface of tray table 3. To this control device 20, medical care chair 1, a plurality of medical devices 4, instrument holder 30, basin unit 2, operation panel 8, an operation monitor 9, a touch panel 9a, and display monitor 6 are connected. Control device 20 includes a main control unit 12, a medical device drive unit 14, an operation monitor control unit 13, a display monitor control unit 15, and a hold-release identification unit 18, as will be described later. Each of these components is configured by a central processing unit (CPU), a read only memory (ROM), a random access memory (RAM) and the like that are mounted on a substrate (not shown).

Medical device 4 is, for example, an instrument for dental care such as an air turbine handpiece, a micro motor handpiece, a scaler, a three-way syringe, and a vacuum syringe. Medical device 4 is not limited to the above components, but may be an intraoral camera, a photopolymerization irradiator, a root canal length measuring instrument, a root canal enlargement instrument, and the like, or may be a non-driving instrument such as a mirror and a syringe.

In medical care apparatus 10, five types of medical devices 4a, 4b, 4c, 4d, and 4e are used. For example, medical devices 4a and 4b each are an air turbine handpiece. Medical device 4c is a micro motor handpiece. Medical device 4d is a scaler. Medical device 4e is a three-way syringe.

Each medical device 4 is held by instrument holder 30. When medical device 4 is removed from instrument holder 30, the held state by instrument holder 30 is released, which leads to a state where medical device 4 is selected (referred to as a selected state).

Each medical device 4 is connected to medical device drive unit 14 included in control device 20. Medical device drive unit 14 drives each medical device 4 based on the user's operation of pressing down foot controller 5. For example, when the user uses his/her foot to press down the switch for driving the air turbine handpiece in foot controller 5, medical device drive unit 14 rotates a cutting tool held at a head portion of the air turbine handpiece. In this way, medical device drive unit 14 has a function of driving medical device 4 based on the user's operation performed on foot controller 5.

Instrument holder 30 serves as a holding member for holding each medical device 4.

Instrument holder 30 is connected to main control unit 12 via hold-release identification unit 18 provided in control device 20. Hold-release identification unit 18 identifies that the state where medical device 4 is held by instrument holder 30 has been released, and then, outputs a signal showing the identification result to main control unit 12. Based on the signal from hold-release identification unit 18, main control unit 12 identifies medical device 4 in the selected state.

As shown in Fig. 1, basin unit 2 is provided on the side portion of medical care chair 1. Basin unit 2 includes a main body unit 2d, a bowl 2a provided with an exhaust port, a cup stand 2b on which a cup is placed, and a water faucet 2c for supplying water to the cup. The patient can gargle with water supplied into the cup from water faucet 2c.

As shown in Fig. 2, basin unit 2 includes a basin control unit 16. Basin control unit 16 is configured to control the flow of water that is to be used in medical care apparatus 10. In this way, basin control unit 16 has a function of controlling the flow of the water used in medical care apparatus 10.

Furthermore, basin unit 2 includes a lighting control unit 17. Lighting control unit 17 is configured to control lighting device 7 to be turned on and off. In this way, lighting control unit 17 has a function of controlling lighting device 7 to be turned on and off.

As shown in Fig. 1, operation monitor 9 is provided on operation panel 8. Operation monitor 9 displays operation images used for operating medical care instruments such as medical care chair 1, each medical device 4, basin unit 2, and lighting device 7. The user can select a desired operation image by pressing touch panel 9a. For example, by selecting an operation image for operating the medical care instrument, the user can operate the desired medical care instrument. Furthermore, by selecting an operation image for setting the function of the medical care instrument, the user can set the function of the desired medical care instrument. Medical care chair 1, each medical device 4, basin unit 2, and lighting device 7 correspond to one embodiment of the "medical care instrument".

Operation monitor 9 is connected to main control unit 12 through operation monitor control unit 13 provided in control device 20. Based on the command from main control unit 12, operation monitor control unit 13 causes operation monitor 9 to display an operation image and the like. Thereby, on operation monitor 9, an operation image and the like are displayed so as to be visible via touch panel 9a from the outside. On the other hand, when the operation image displayed on operation monitor 9 is selected by the user, main control unit 12 controls seat drive unit 11, medical device drive unit 14, basin control unit 16, lighting control unit 17, and the like based on the selected operation image.

Display monitor 6 is located differently from operation monitor 9. In the present embodiment, as shown in Fig. 1, operation monitor 9 is disposed on operation panel 8 provided in tray table 3 while display monitor 6 is disposed at the arm extending upward from tray table 3.

Display monitor 6 has a screen that is greater in display area than the screen provided in operation monitor 9. Thus, display monitor 6 can display an image that cannot be displayed in the display area of operation monitor 9. In other words, display monitor 6 can display information that is greater in amount than the information displayed on operation monitor 9. Also, display monitor 6 can implement a display that cannot be implemented by operation monitor 9.

For example, display monitor 6 displays: an image of a patient's chart; an image showing the position at the tip end of a cutting tool inside a root canal; an image inside the oral cavity taken by an intraoral camera; and the like. Furthermore, display monitor 6 can display the information associated with the operation image displayed on operation monitor 9. In other words, when operation monitor 9 displays the operation image, display monitor 6 displays the associated image associated with the operation image. The associated image is different from the operation image displayed on operation monitor 9 and includes information that cannot be displayed on operation monitor 9. For example, display monitor 6 displays, as associated images: a setting image for medical device 4 associated with the operation image corresponding to medical device 4; a setting image for lighting device 7 associated with the operation image corresponding to lighting device 7; an image showing the current allocation state and associated with the operation image corresponding to each switch on foot controller 5; and the like. In addition to the above-described images, display monitor 6 may display the same image as that displayed on operation monitor 9.

Display monitor 6 is connected to main control unit 12 through display monitor control unit 15 provided in control device 20. Based on the command from main control unit 12, display monitor control unit 15 causes display monitor 6 to display: an image that cannot be displayed in the display area of operation monitor 9; an associated image associated with the operation image; and the like.

Operation monitor 9 corresponds to one embodiment of the "first display unit". Display monitor 6 corresponds to one embodiment of the "second display unit". Main control unit 12 and display monitor control unit 15 correspond to one embodiment of the "display control unit". The "display control unit" is configured to include operation monitor control unit 13 for operation monitor 9 and include display monitor control unit 15 for display monitor 6, but may be configured to include one monitor control unit for both of operation monitor 9 and display monitor 6.

### [Display Examples of Operation Monitor and Display Monitor]

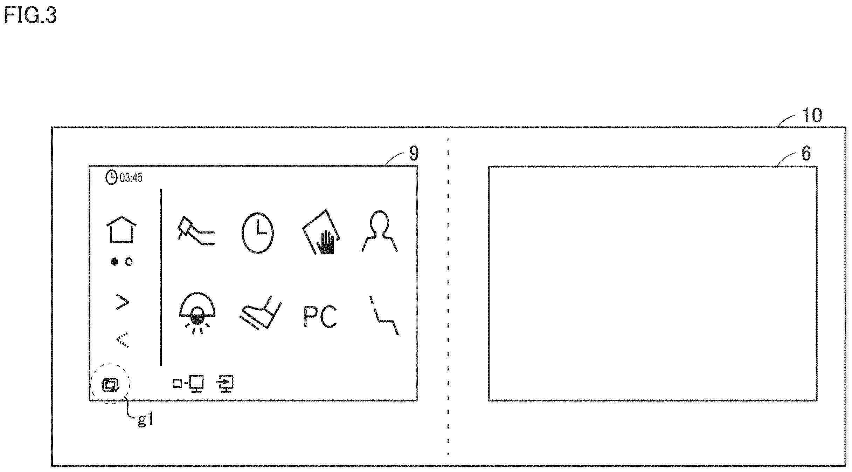

Referring to Figs. 3 to 13, display examples of operation monitor 9 and display monitor 6 will be hereinafter described. As shown in Figs. 3 to 13, operation monitor 9 displays an operation image selected by the user. Operation monitor 9 is configured to display an operation image so as to be easily visible to the user (hereinafter also referred to as "illuminate") and so as to be hardly visible to the user (hereinafter also referred to as "unilluminate"). Furthermore, display monitor 6 can display an associated image that is associated with the operation image displayed on operation monitor 9.

### (Menu Screen in Non-Associated Mode)

Fig. 3 is a diagram for illustrating display examples of operation monitor 9 and display monitor 6 in the non-associated mode. In the present embodiment, the user can select one of: the associated mode in which an associated image is displayed on display monitor 6; and the non-associated mode in which an associated image is not displayed on display monitor 6. Fig. 3 shows an example in which the non-associated mode is selected. In this example, a menu screen is displayed on operation monitor 9 while an associated image is not displayed on display monitor 6. Even if the non-associated mode is selected, the image of the patient's chart and the like other than the associated image may be displayed on display monitor 6.

As shown in Fig. 3, eight operation images are displayed in the center and right-side area on operation monitor 9 in such a manner that these operation images are separately arranged on two upper and lower stages. For example, starting from the left on the upper stage, operation monitor 9 displays an operation image for selecting and setting each medical device 4, an operation image for setting the time, an operation image for starting the maintenance mode described later, and an operation image for performing the user setting, sequentially in this order. By selecting the operation image used for performing the user setting, the user can allocate the function of driving medical device 4, the function of changing the posture of medical care chair 1 and the like to corresponding switches on foot controller 5. Furthermore, starting from the left on the lower stage, operation monitor 9 displays an operation image for setting lighting device 7, an operation image for allocating a prescribed function to each switch on foot controller 5, an operation image for setting a personal computer (PC), and an operation image for setting the posture of medical care chair 1, sequentially in this order. Also on the left side of operation monitor 9, starting from the top, operation monitor 9 displays an operation image for switching to a home screen, an operation image for switching between pages, and an operation image gl for switching to the associated mode, sequentially in this order. The user can select each of the operation images. Below the operation image for switching to a home screen, there are a black dot mark and a white circle mark arranged side by side, which means that operation monitor 9 currently displays the first page of two pages that can be displayed. When the operation image for switching between pages is operated, the first page is switched to the second page; and the black dot mark is changed into a white circle mark while the white circle mark is changed into a black dot mark, thereby indicating that the changed page shows the second page of two pages that can be displayed.

When the non-associated mode is selected, display monitor 6 does not display an associated image while operation monitor 9 displays a menu screen shown in Fig. 3.

### (Menu Screen in Associated Mode)

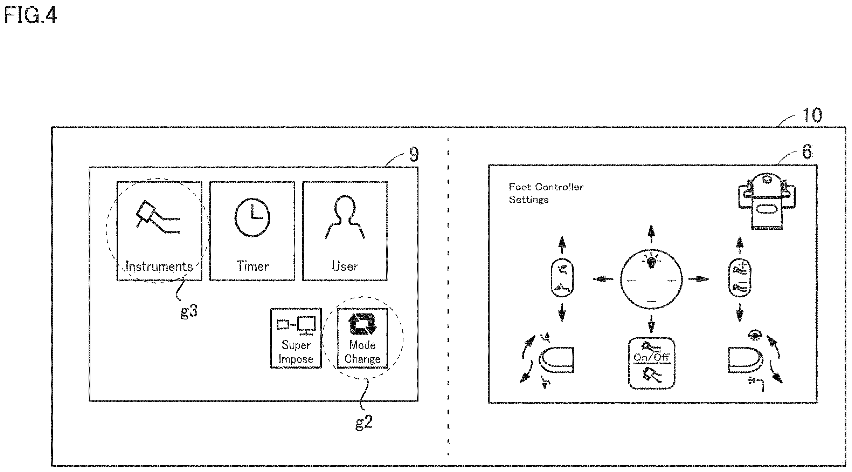

Fig. 4 is a diagram for illustrating display examples of operation monitor 9 and display monitor 6 in the associated mode. When the user selects an operation image g1 for switching to the associated mode on the menu screen shown in Fig. 3, the mode is switched to the associated mode. Then, operation monitor 9 displays a menu screen shown in Fig. 4.

As shown in Fig. 4, three operation images are displayed on the upper stage of operation monitor 9. For example, starting from the left on the upper stage, operation monitor 9 displays an operation image g3 for selecting and setting each medical device 4, an operation image for setting the time, and an operation image for performing the user setting. Furthermore, starting from the left on the lower stage, operation monitor 9 displays an image for using a superimposing function, and an operation image g2 for switching to the non-associated mode. The superimposing function is to display images in such a manner that an image displayed on operation monitor 9 is superimposed on an image displayed on display monitor 6. For example, by using the superimposing function, the operation image displayed on operation monitor 9 is superimposed on the image such as a chart displayed on display monitor 6.

When the associated mode is selected, display monitor 6 displays, as an associated image, an image showing the current state of allocation to each switch on foot controller 5 while operation monitor 9 displays a menu screen shown in Fig. 4.

When the user selects operation image g2 for switching to the non-associated mode, the mode is switched to the non-associated mode. Then, on operation monitor 9, the screen is again switched to the menu screen shown in Fig. 3.

In this way, by selecting operation image g1 for switching to the associated mode, the user can cause display monitor 6 to display an associated image. Thereby, it becomes possible to improve the convenience at the time when the user selects an operation image. On the other hand, when an associated image does not need to be displayed, the user selects operation image g2 for switching to the non-associated mode, so that the user can disallow display monitor 6 to display an associated image.

### (Selection Screen for Each Medical Device)

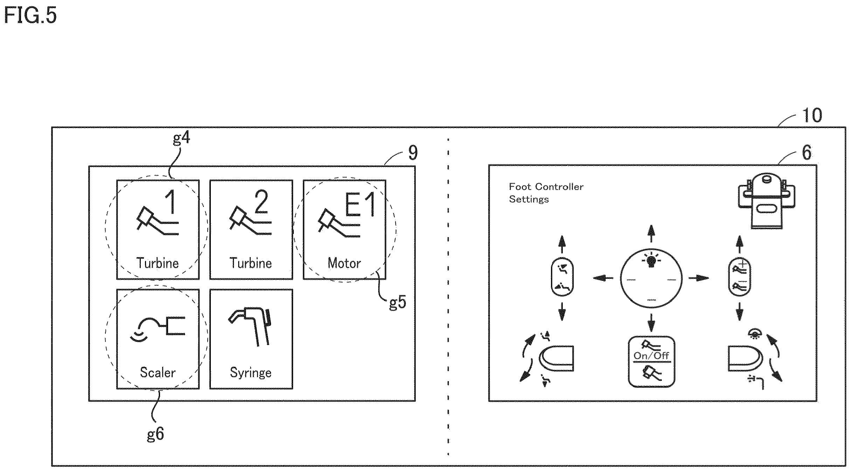

Fig. 5 is a diagram for illustrating display examples of operation monitor 9 and display monitor 6 in the associated mode. Particularly, Fig. 5 shows an example in which operation monitor 9 displays a selection screen for each medical device 4 in the associated mode.

When the user selects operation image g3 for selecting and setting each medical device 4 on the menu screen shown in Fig. 4, the screen is switched on operation monitor 9 to the selection screen for each medical device 4 as shown in Fig. 5.

The selection screen shown in Fig. 5 displays five operation images that are arranged separately on two upper and lower stages. For example, starting from the left on the upper stage, operation monitor 9 displays an operation image g4 for selecting an air turbine handpiece corresponding to medical device 4a, an operation image for selecting an air turbine handpiece corresponding to medical device 4b, and an operation image g5 for selecting a micro motor handpiece corresponding to medical device 4c, sequentially in this order. Furthermore, starting from the left on the lower stage, operation monitor 9 displays an operation image g6 for selecting a scaler corresponding to medical device 4d, and an operation image for selecting a three-way syringe corresponding to medical device 4e, sequentially in this order. It is needless to say that the configuration having five operation images displayed separately on two stages is merely by way of example. However, depending on the specifications of the apparatus, there may be other configurations, for example, in which three operation images may be displayed on one stage, or six operation images may be displayed in such a manner that one group of three operation images is arranged on the first stage while the other group of remaining three operation images is arranged on the second stage.

By selecting each operation image, the user can select medical device 4 corresponding to the selected operation image so as to bring medical device 4 into the selected state. Then, the user can cause operation monitor 9 to display the setting screen for the selected medical device 4. Furthermore, also by removing medical device 4 from instrument holder 30 and releasing medical device 4 from holding by instrument holder 30, the user can also bring medical device 4 into the selected state. Also in this case, the setting screen for the selected medical device 4 is displayed on operation monitor 9.

On the other hand, while operation monitor 9 displays the selection screen shown in Fig. 5, display monitor 6 displays, as an associated image, an image showing the current state of allocation to each switch on foot controller 5.

### (Setting Screen for Air Turbine Handpiece)

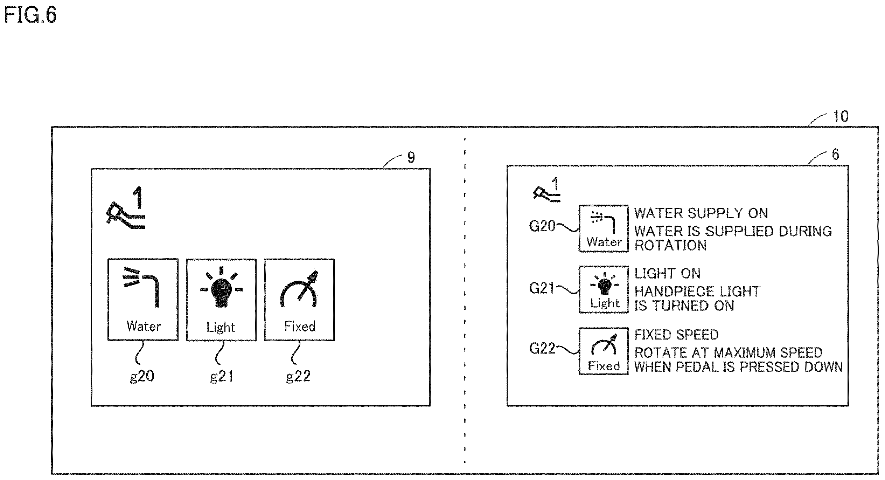

Fig. 6 is a diagram for illustrating display examples of operation monitor 9 and display monitor 6 in the associated mode. Particularly, Fig. 6 shows an example in which operation monitor 9 displays the setting screen for the air turbine handpiece in the associated mode.

When the user selects operation image g4 for selecting an air turbine handpiece on the selection screen for each medical device 4 shown in Fig. 5, or when the user removes the air turbine handpiece from instrument holder 30, the user can bring the air turbine handpiece into the selected state. In this case, as shown in Fig. 6, the screen is switched to the setting screen for the air turbine handpiece on operation monitor 9. Display monitor 6 displays an image about each setting for the air turbine handpiece as an associated image.

The setting screen for the air turbine handpiece shown in Fig. 6 displays: an operation image g20 for setting a handpiece ("HP": the same as above) water supply unit provided at the tip end of the air turbine handpiece; an operation image g21 for setting an HP light provided at the tip end of the air turbine handpiece; and an operation image g22 for switching the rotation speed mode of the air turbine handpiece.

When the user selects operation image g20, water supply by the HP water supply unit can be switched to be started (ON state) and stopped (OFF state). In the example shown in Fig. 6, water supply by the HP water supply unit is in the ON state and operation image g20 is illuminated. When the user selects operation image g21, the HP light can be switched to be turned on and off. In the example shown in Fig. 6, the HP light is in the ON state and operation image g21 is illuminated. When the user selects operation image g22, the rotation speed of the air turbine handpiece can be switched between a fixed speed mode (the mode in which the cutting tool at the tip end rotates at a predetermined fixed speed) and a variable speed mode (for example, the mode in which the cutting tool at the tip end rotates at a speed in accordance with the amount of pressing down foot controller 5 by the user). In the example shown in Fig. 6, the rotation speed of the air turbine handpiece is set in the fixed speed mode, and operation image g22 shows the term "Fixed" to represent that the fixed speed mode is being selected.

In this case, however, only operation images g20 to g22 shown in Fig. 6 cannot allow the user to sufficiently understand as to what kind of function each operation image specifically represents. Accordingly, while operation monitor 9 displays the screen shown in Fig. 6, display monitor 6 displays, as associated images, images G20, G21 and G22 including information required when the user selects operation images g20, g21 and g22, respectively.

For example, image G20 is associated with operation image g20, and represents the function shown by operation image g20 and the currently selected ON/OFF state of the HP water supply unit. In the example shown in Fig. 6, image G20 is displayed, which represents that the HP water supply unit is in the ON state and that water is supplied during rotation of the air turbine handpiece. Image G21 is associated with operation image g21, and represents the function shown by operation image g21 and the currently selected ON/OFF state of the HP light. In the example shown in Fig. 6, image G21 is displayed, which represents that the HP light is in the ON state and that the HP light is turned on during rotation of the air turbine handpiece. Image G22 is associated with operation image g22, and represents the function shown by operation image g22 and the currently selected fixed speed mode/variable speed mode of the rotation speed of the air turbine handpiece. In the example shown in Fig. 6, image G22 is displayed, which represents: that the rotation speed of the air turbine handpiece is set in the fixed speed mode; and that the cutting tool at the tip end rotates at the maximum speed in accordance with the performance of the air turbine handpiece when the pedal is pressed down.

### (Setting Screen for Micro Motor Handpiece)

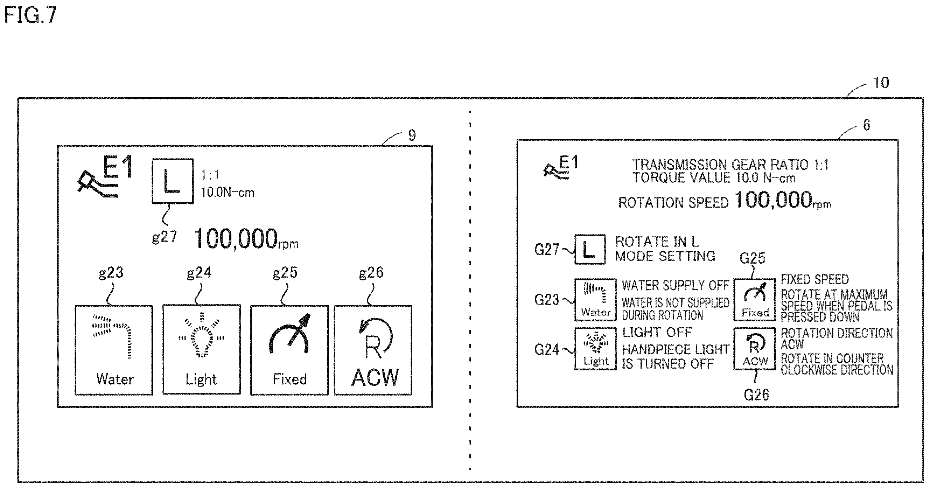

Fig. 7 is a diagram for illustrating display examples of operation monitor 9 and display monitor 6 in the associated mode. Particularly, Fig. 7 shows an example in which operation monitor 9 displays a setting screen for the micro motor handpiece in the associated mode.

When the user selects operation image g5 for selecting a micro motor handpiece on the selection screen for each medical device 4 shown in Fig. 5, or when the user removes the micro motor handpiece from instrument holder 30, the micro motor handpiece can be brought into the selected state. In this case, as shown in Fig. 7, the screen is switched to the setting screen for the micro motor handpiece on operation monitor 9. Display monitor 6 displays the image about each setting for the micro motor handpiece as an associated image.

The setting screen for the micro motor handpiece shown in Fig. 7 displays: an operation image g23 for setting the HP water supply unit provided at the tip end of the micro motor handpiece; an operation image g24 for setting the HP light provided at the tip end of the micro motor handpiece; an operation image g25 for switching the rotation speed mode of the micro motor handpiece; an operation image g26 for switching the rotation direction mode of the micro motor handpiece; and an operation image g27 for switching the rotation speed range of the micro motor handpiece.

When the user selects operation image g23, water supply by the HP water supply unit can be switched to be started (ON state) and stopped (OFF state). In the example shown in Fig. 7, water supply by the HP water supply unit is in the OFF state and operation image g23 is unilluminated. When the user selects operation image g24, the HP light can be switched to be turned on and off. In the example shown in Fig. 7, the HP light is in the OFF state and operation image g24 is unilluminated. When the user selects operation image g25, the rotation speed of the micro motor handpiece can be switched between the fixed speed mode and the variable speed mode. In the example shown in Fig. 7, the rotation speed of the micro motor handpiece is set in the fixed speed mode, and operation image g25 shows the term "Fixed" to represent that the fixed speed mode is being selected. When the user selects operation image g26, the rotation direction of the micro motor handpiece can be switched between the normal rotation mode (the mode in which the cutting tool at the tip end rotates in the clockwise direction toward the tip end of the tool) and the reverse rotation mode (the mode in which the cutting tool at the tip end rotates in the counter-clockwise direction toward the tip end of the tool). In the example shown in Fig. 7, the rotation direction of the micro motor handpiece is set in the reverse rotation mode, and operation image g26 shows the term "ACW (Anti-ClockWise)" to represent that the reverse rotation mode is being selected. When the user selects operation image g27, the rotation speed range of the micro motor handpiece can be selected from among the ultra low (UL) mode, the low (L) mode, the middle (M) mode, and the high (H) mode. In the example shown in Fig. 7, the rotation speed range of the micro motor handpiece is set in the L mode, and operation image g27 shows that the L mode is being selected.

In this case, only operation images g23 to g27 shown in Fig. 7 may not allow the user to sufficiently understand as to what kind of function each operation image specifically represents. Thus, while operation monitor 9 displays the screen shown in Fig. 7, display monitor 6 displays, as associated images, images G23, G24, G25, G26, and G27 including information required when the user presses operation images g23, g24, g25, g26, and g27, respectively.

For example, image G23 is associated with operation image g23, and represents the function shown by operation image g23 and the currently selected ON/OFF state of the HP water supply unit. In the example shown in Fig. 7, image G23 is displayed, which represents that the HP water supply unit is in the OFF state, and that water is not supplied during rotation of the micro motor handpiece. Image G24 is associated with operation image g24, and represents the function shown by operation image g24 and the currently selected ON/OFF state of the HP light. In the example shown in Fig. 7, image G24 is displayed, which represents that the HP light is in the OFF state and that the HP light is turned off during rotation of the micro motor handpiece. Image G25 is associated with operation image g25, and represents the function shown by operation image g25 and the currently selected fixed speed mode/variable speed mode of the rotation speed of the micro motor handpiece. In the example shown in Fig. 7, image G25 is displayed, which represents that the rotation speed of the micro motor handpiece is set in the fixed speed mode, and that the cutting tool at the tip end rotates at the maximum speed in accordance with the performance of the micro motor handpiece when the pedal is pressed down. Image G26 is associated with operation image g26, and represents the function shown by operation image g26 and the currently selected normal rotation mode/reverse rotation mode about the rotation direction of the micro motor handpiece. In the example shown in Fig. 7, image G26 is displayed, which represents that the rotation direction of the micro motor handpiece is set in the reverse rotation mode, and that the cutting tool at the tip end rotates in the counter clockwise direction toward the tip end of the tool. Image G27 is associated with operation image g27, and represents the function shown by operation image g27 and the currently selected rotation speed range of the micro motor handpiece. In the example shown in Fig. 7, image G27 is displayed, which represents that the micro motor handpiece rotates at the rotation speed in the L mode setting.

### (Setting Screen for Scaler)

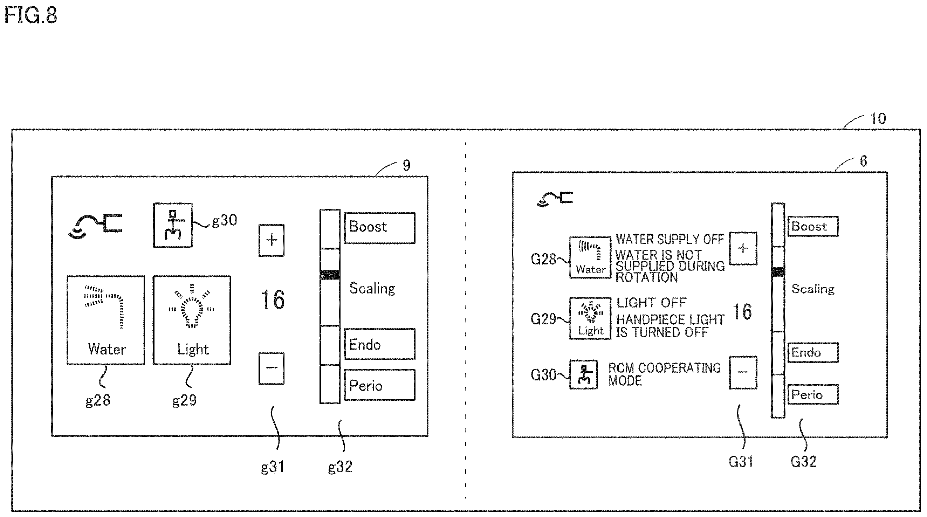

Fig. 8 is a diagram for illustrating display examples of operation monitor 9 and display monitor 6 in the associated mode. Particularly, Fig. 8 shows an example in which operation monitor 9 displays the setting screen for the scaler in the associated mode.

When the user selects operation image g6 for selecting a scaler on the selection screen for each medical device 4 shown in Fig. 5, or when the user removes the scaler from instrument holder 30, the scaler can be brought into the selected state. In this case, as shown in Fig. 8, the screen is switched to the setting screen for the scaler on operation monitor 9. Furthermore, display monitor 6 displays an image about each setting for the scaler as an associated image.

The setting screen for the scaler shown in Fig. 8 displays an operation image g28 for setting the HP water supply unit provided at the tip end of the scaler; an operation image g29 for setting the HP light provided at the tip end of the scaler; an operation image g30 for setting the root canal measurement (RCM) cooperating mode (the mode in which a treatment can be conducted using the scaler while measuring the root canal length); and an operation image g31 for switching the vibration level of the scaler.

When the user selects operation image g28, water supply by the HP water supply unit can be switched to be started (ON state) and stopped (OFF state). In the example shown in Fig. 8, water supply by the HP water supply unit is in the OFF state, and operation image g28 is unilluminated. When the user selects operation image g29, the HP light can be switched to be turned on and off. In the example shown in Fig. 8, the HP light is in the OFF state and operation image g29 is unilluminated. When the user selects operation image g30, the RCM cooperating mode can be switched to be on and off. In the example shown in Fig. 8, the RCM cooperating mode is in the ON state and operation image g30 is illuminated. When the user selects operation image g31, the vibration level of the scaler can be switched. For example, when the user selects an operation image of "+" in operation image g31, the vibration level of the scaler can be raised. When the user selects an operation image of"-", the vibration level of the scaler can be lowered. The set vibration level of the scaler is represented by an image g32.

In this case, however, only operation images g28 to g31 shown in Fig. 8 may not allow the user to sufficiently understand as to what kind of function each operation image specifically represents. Thus, while operation monitor 9 displays the screen shown in Fig. 8, display monitor 6 displays, as associated images, images G28, G29, G30, G31, and G32 including information required when the user presses operation images g28, g29, g30, and g31.

For example, image G28 is associated with operation image g28, and represents the function shown by operation image g28 and the currently selected ON/OFF state of the HP water supply unit. In the example shown in Fig. 8, image G28 is displayed, which represents that the HP water supply unit is in the OFF state and that water is not supplied during driving of the scaler. Image G29 is associated with operation image g29, and represents the function shown by operation image g29 and the currently selected ON/OFF state of the HP light. In the example shown in Fig. 8, image G29 is displayed, which represents that the HP light is in the OFF state and that the HP light is turned off during driving of the scaler. Image G30 is associated with operation image g30, and represents the function shown by operation image g30 and the currently selected ON/OFF state in the RCM cooperating mode. In the example shown in Fig. 8, image G30 is displayed, which represents that the RCM cooperating mode is currently in the ON state. Image G31 is associated with operation image g31, and represents the vibration level of the scaler set by operation image g31. Image G32 represents the currently selected vibration level of the scaler, as with operation image g32.

In this way, when the user selects the operation image corresponding to the desired medical device 4 on the selection screen for each medical device 4 shown in Fig. 5, the setting screen corresponding to medical device 4 selected by the user is displayed on operation monitor 9. Then, according to selection of the operation image by the user, the screen on display monitor 6 is also switched to the screen corresponding to medical device 4 selected by the user. In other words, in accordance with the type of the selected medical device 4, the associated image displayed on display monitor 6 is also switched. Thereby, the user can confirm that medical care apparatus 10 has recognized that medical device 4 has been selected by the user's selection of the operation image.

Furthermore, while operation monitor 9 displays the setting screen for each medical device 4, display monitor 6 displays the associated image associated with the operation image displayed on operation monitor 9. For example, while operation monitor 9 displays the setting screen for the air turbine handpiece shown in Fig. 6, display monitor 6 displays detailed information as an associated image associated with each of the operation images about: a water supply function by the HP water supply unit as a function provided in the air turbine handpiece; an illuminating function by the HP light; and a function of changing the rotation speed of the air turbine handpiece. For example, as detailed information, image G20 represents that water is supplied during rotation of the air turbine handpiece; image G21 represents that the HP light is turned on during rotation of the air turbine handpiece; and image G22 represents that the cutting tool at the tip end rotates at the maximum speed in accordance with the performance of the air turbine handpiece when the pedal is pressed down. These pieces of detailed information are required when the user sets the function of medical care apparatus 10. Thereby, the user can obtain sufficient information required when the user selects an operation image.

### (Screen in Maintenance Mode)

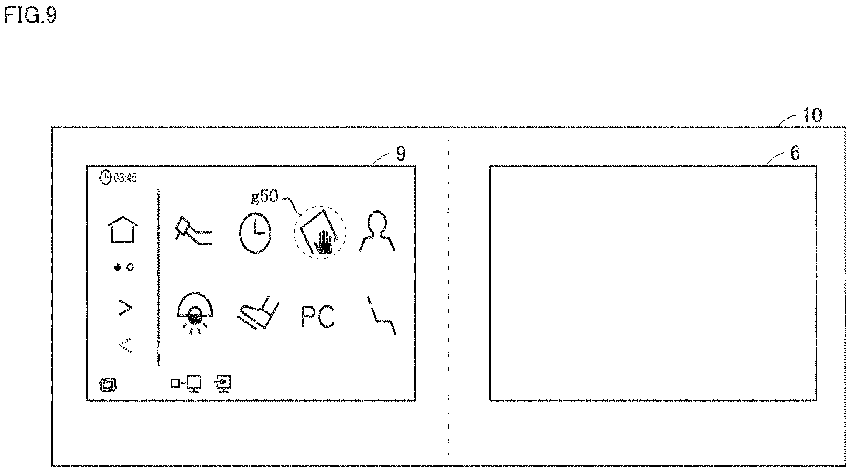

Figs. 9 to 13 each are a diagram for illustrating display examples of the operation monitor and the display monitor in the maintenance mode. The maintenance mode is implemented, for example, when residual water accumulated in medical device 4 serving as an instrument for dental care and residual water accumulated inside medical care apparatus 10 are discharged (also referred to as "flushed"), and at the same time, water is supplied into assistant instruments such as a saliva ejector (saliva discharge tube) and a vacuum chip for assisting medical care, thereby cleaning these instruments. Also, the maintenance mode is implemented, for example, when water pipes and various instruments inside medical care apparatus 10 are filled with a cleaning liquid, thereby keeping the water pipe clean (also referred to as a pipe cleaning system).

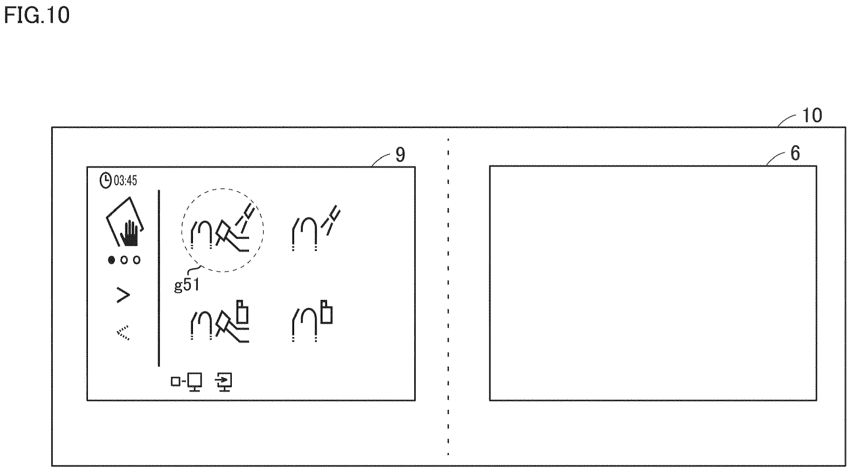

As shown in Fig. 9, when the user selects an operation image g50 for starting the maintenance mode from the menu screen displayed on operation monitor 9, the screen is switched to the maintenance mode selection screen as shown in Fig. 10.

The screen shown in Fig. 10 displays: an operation image g51 for starting the automatic cleaning mode in which discharge of residual water from each instrument and cleaning of each instrument are automatically performed; an operation image for starting the pipe cleaning system using a cleaning liquid; and the like.

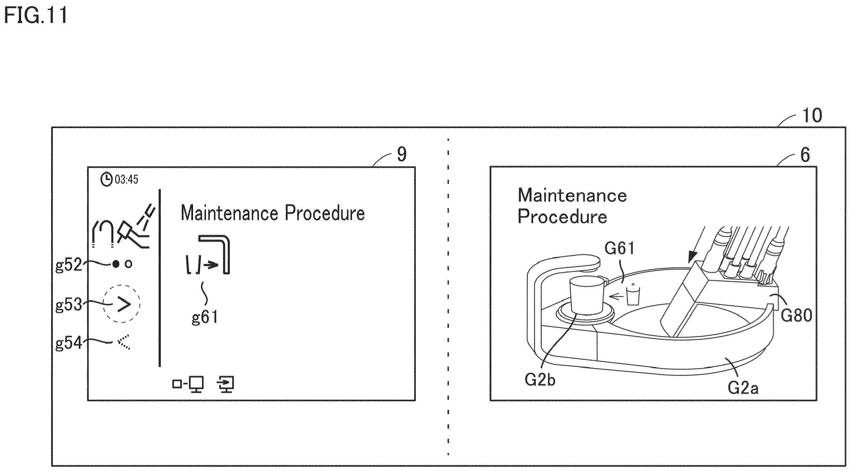

When the user selects operation image g51 on the maintenance mode selection screen shown in Fig. 10, the screen is switched on operation monitor 9 to the screen showing the process procedure in the maintenance mode, as shown in Fig. 11. Furthermore, display monitor 6 displays an image specifically showing the process procedure in the maintenance mode as an associated image. In the present embodiment, when the maintenance mode is selected, the associated mode is forcibly selected to thereby cause display monitor 6 to display an associated image. Even if the maintenance mode is selected, the user may be able to select the associated mode or the non-associated mode.

In this case, the detailed process procedure in the maintenance mode is normally described in the instruction manual of medical care apparatus 10. Thus, it is difficult for the user to completely understand the process procedure only from the information obtained from the image displayed on operation monitor 9. Accordingly, as described below, while operation monitor 9 displays an image showing the process procedure in the maintenance mode, display monitor 6 displays, as an associated image, an image illustrating the detailed process procedure in the maintenance mode as explained in the instruction manual so as to be in cooperation with the image displayed on operation monitor 9.

As shown in Fig. 11, operation monitor 9 displays an image g61 showing a symbol to represent that a cup is placed on cup stand 2b. Operation monitor 9 also displays an image 52 showing the current page, operation images 53 and 54 for switching the page, and the like.

On the other hand, while operation monitor 9 displays the screen shown in Fig. 11, display monitor 6 displays, as associated images, an image G2a showing bowl 2a of basin unit 2, an image G2b showing cup stand 2b, and an image G80 showing a flushing apparatus for performing automatic flushing. Then, while utilizing such image G2a showing bowl 2a of basin unit 2, display monitor 6 displays an image G61 for urging the user to place a cup on cup stand 2b.

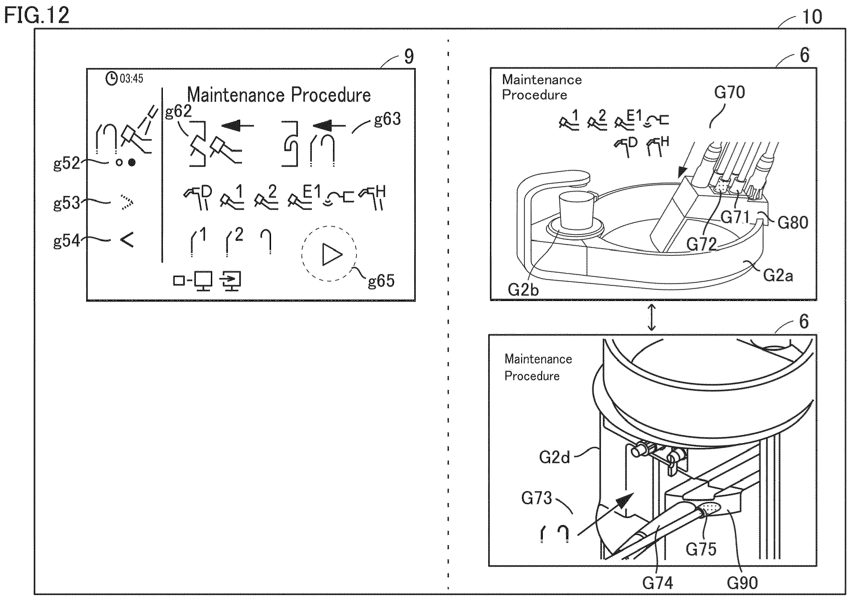

When the user selects an operation image g53 on the screen shown in Fig. 11, the page is switched to the next page showing the maintenance procedure as shown in Fig. 12.

As shown in Fig. 12, operation monitor 9 displays: an image g62 showing a symbol to represent that medical device 4 is inserted into the flushing apparatus; an image g63 showing a symbol to represent that the assistant instrument is inserted into the cleaning apparatus; an operation image g65 for starting automatic flushing; and the like. When all of medical devices 4 and assistant instruments are not inserted into the flushing apparatus and the cleaning apparatus, operation image g65 for starting automatic flushing is unilluminated, so that selection of operation image g65 by the user is not detected.

On the other hand, while operation monitor 9 displays the screen shown in Fig. 12, display monitor 6 displays, as associated images, an image G2a showing bowl 2a of basin unit 2, an image G2b showing cup stand 2b, and an image G80 showing a flushing apparatus attached to bowl 2a. Then, while utilizing such image G2a showing bowl 2a of basin unit 2, display monitor 6 displays an image G70 for urging the user to insert each medical device 4 into the flushing apparatus attached to bowl 2a. When the user does not insert medical device 4 into the flushing apparatus, the image corresponding to this medical device 4 is displayed in white color (for example, see an image G71). When the user inserts medical device 4 into the flushing apparatus, the color of the image corresponding to this medical device 4 changes from white to another color (for example, see an image G72).

Furthermore, while the screen shown in Fig. 12 is displayed on operation monitor 9, the screen is switched in each prescribed time period (for example, in every five seconds) on display monitor 6. Display monitor 6 shown on the upper side displays, as associated images, an image G2d showing main body unit 2d of basin unit 2, and an image G90 showing the cleaning apparatus provided inside main body unit 2d. Then, while utilizing such image G2d showing main body unit 2d of basin unit 2, display monitor 6 displays an image G73 for urging the user to insert the assistant instrument into the cleaning apparatus, which is provided inside main body unit 2d and into which water is poured. When the user does not insert the assistant instrument into the cleaning apparatus, the image corresponding to the assistant instrument is displayed in white color (for example, see an image G74). When the user inserts the assistant instrument into the cleaning apparatus, the color of the image corresponding to the assistant instrument changes from white to another color (for example, see an image G75).

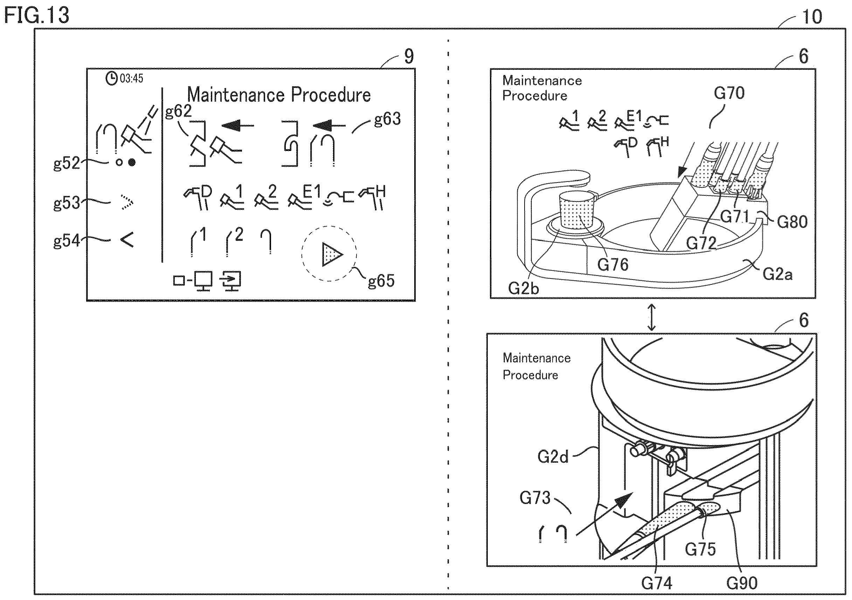

As shown in Fig. 13, when the user places a cup on cup stand 2b, the color of the image of the cup changes from white to another color (for example, see an image G76) on display monitor 6. Furthermore, when the user inserts all of medical devices 4 and assistant instruments into the flushing apparatus and the cleaning apparatus, the colors of the images of all of medical devices 4 and assistant instruments change from white to another color on display monitor 6. Furthermore, operation image g65 for starting automatic flushing is illuminated on operation monitor 9, so that selection of this operation image g65 by the user can be detected. When the user selects operation image g65 in such a state, automatic flushing and water cleaning are started.

Thus, while operation monitor 9 displays the operation image showing the process procedure in the maintenance mode, display monitor 6 displays an associated image associated with the operation image displayed on operation monitor 9. For example, as shown in Fig. 11, while operation monitor 9 displays image g61 showing a symbol to represent that a cup is placed on cup stand 2b, display monitor 6 displays image G61 for urging the user to place a cup on cup stand 2b, so as to visually facilitate understanding by the user. Also as shown in Fig. 12, while operation monitor 9 displays image g62 showing a symbol to represent that medical device 4 is inserted into the flushing apparatus and an image g63 showing a symbol to represent that the assistant instrument is inserted into the cleaning apparatus, display monitor 6 displays image G70 for urging the user to insert each medical device 4 into the flushing apparatus and image G73 for urging the user to insert the assistant instrument into the cleaning apparatus, so as to visually facilitate understanding by the user. In this way, display monitor 6 displays the information required when the user performs maintenance of medical device 4 and the assistant instrument, so as to visually facilitate understanding by the user. Accordingly, the user can confirm the information required when the user performs maintenance of medical device 4 and the assistant instrument.

Also as shown in Fig. 12, each time the user inserts medical device 4 into the flushing apparatus and each time the user inserts the assistant instrument into the cleaning apparatus, the image displayed on display monitor 6 is switched. Then, as shown in Fig. 13, when all of medical devices 4 and assistant instruments are inserted into the flushing apparatus and the cleaning apparatus, operation image g65 for starting automatic cleaning is illuminated on operation monitor 9, and the user selects this operation image g65, thereby starting automatic cleaning. In this way, since the associated image displayed on display monitor 6 is switched in each progress of the maintenance of medical device 4 and the assistance instrument performed by the user (the flushing and cleaning operation), the user can recognize the progress degree of the maintenance operation for medical device 4 and the assistant instrument.

### [Display Control Process]

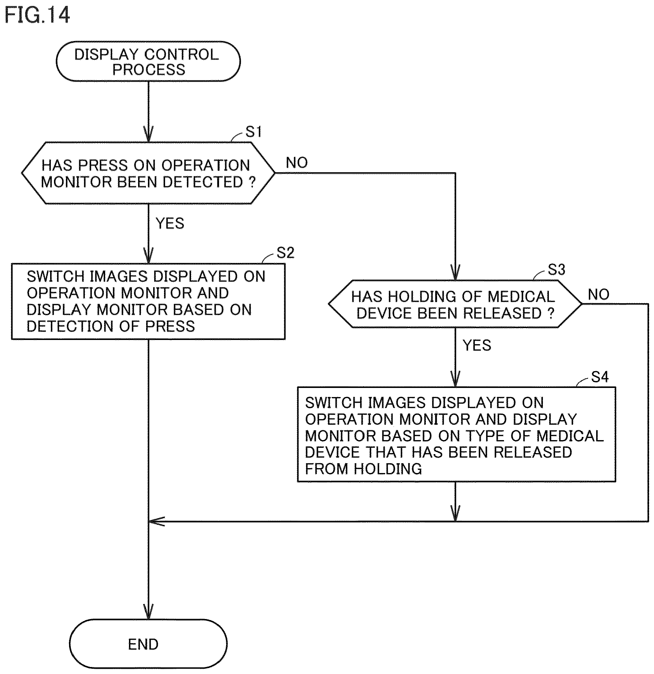

The display control process performed by medical care apparatus 10 will be hereinafter described with reference to Fig. 14. Fig. 14 is a flowchart for illustrating a display control process performed by medical care apparatus 10. The display control process shown in Fig. 14 is performed periodically (for example, for every 100 msec) by main control unit 12 included in medical care apparatus 10.

As shown in Fig. 14, medical care apparatus 10 determines whether a press by the user on operation monitor 9 has been detected or not (S1). When medical care apparatus 10 detects a press on operation monitor 9 (YES in S1), it switches the image displayed on operation monitor 9 and the image displayed on display monitor 6 based on this detection of the press (S2). Specifically, main control unit 12 controls operation monitor control unit 13 to illuminate and unilluminate the operation image on operation monitor 9. Furthermore, main control unit 12 controls operation monitor control unit 13 to change the image displayed on operation monitor 9 such that the screen is switched or the page is switched. Furthermore, main control unit 12 controls display monitor control unit 15 to cause display monitor 6 to display the associated image associated with the operation image displayed on operation monitor 9. Then, medical care apparatus 10 ends the present routine.

On the other hand, when medical care apparatus 10 does not detect a press on operation monitor 9 (NO in S1), it determines whether one of medical devices 4 has been released or not from holding by instrument holder 30 (S3). Specifically, main control unit 12 determines whether the signal for identifying that one of medical devices 4 has been released from holding by instrument holder 30 has been received or not from hold-release identification unit 18.

When one of medical devices 4 has not been released from holding by instrument holder 30 (NO in S3), medical care apparatus 10 ends the present routine. On the other hand, when one of medical devices 4 has been released from holding by instrument holder 30 (YES in S3), medical care apparatus 10 switches the operation image displayed on operation monitor 9 and the associated image displayed on display monitor 6 based on the type of medical device 4 that has been released from holding (S4). Specifically, main control unit 12 controls operation monitor control unit 13 to switch the screen on operation monitor 9 to a screen corresponding to medical device 4 that has been released from holding, thereby causing the switched screen to show the operation image corresponding to this medical device 4. Furthermore, main control unit 12 controls display monitor control unit 15 to cause display monitor 6 to display the associated image associated with the operation image displayed on operation monitor 9. Then, medical care apparatus 10 ends the present routine.

As described above, when medical care apparatus 10 performs the display control process, the associated image associated with the operation image displayed on operation monitor 9 can be displayed on display monitor 6 that is greater in display area than operation monitor 9. Thereby, it becomes possible to improve the convenience at the time when the user operates an operation image.

### [Modifications]

The present invention is not limited to the above-described embodiments, but various modifications and applications thereof can be implemented. The following is an explanation about modifications applicable to the present invention.

In the present embodiment, medical care apparatus 10 includes one operation monitor 9, but medical care apparatus 10 may include a plurality of operation monitors 9. Also in the present embodiment, medical care apparatus 10 includes one display monitor 6, but medical care apparatus 10 may include a plurality of display monitors 6. In the configuration as described above, the associated image associated with the operation image displayed on one of operation monitors 9 may be displayed on one of display monitors 6, or the associated image associated with the operation image displayed on one of operation monitors 9 may be displayed on every display monitor 6.

Also, without being limited to a component having a physical screen like display monitor 6, a component like an eyeglasses-type display unit may be provided, which is worn by the user to thereby allow this user to view the display area greater than operation monitor 9 through lenses. Even in the case of such a wearable-type display unit, the associated image associated with the operation image displayed on operation monitor 9 may be displayed on the display area that is visible to the user.

Operation monitor 9 may display an operation image operated by the user when an abnormality occurs in medical care apparatus 10. Also, display monitor 6 may display an image including the information for notifying the user about occurrence of the abnormality as an associated image associated with the operation image. For example, when an abnormality occurs in medical care apparatus 10, operation monitor 9 displays the operation image for notifying the user about the occurrence of the abnormality and the type of the occurred abnormality. Then, when the user selects the operation image, display monitor 6 may display the image including the detailed information about the occurred abnormality such as occurrence of the abnormality and the type of the occurred abnormality. In this way, by selecting the operation image displayed on operation monitor 9, the user can cause display monitor 6 to display an image including the detailed information about the occurred abnormality, and also can confirm occurrence of the abnormality in detail.

Medical care apparatus 10 may be connected to the Internet through the line with secured safety, and may obtain information such as valuable notifications from the outside. Furthermore, operation monitor 9 may display the operation image operated by the user when the user obtains information from the outside. Also, display monitor 6 may display an image including the information obtained from the outside as an associated image associated with this operation image. For example, when medical care apparatus 10 obtains information from the outside, operation monitor 9 may display the operation image operated by the user when the user obtains the information from the outside. Then, when the user selects this operation image, display monitor 6 may display an image including valuable notification information obtained from the outside (for example, information about a new product from a manufacturer, update information about software, and the like). In this way, by selecting the operation image displayed on operation monitor 9, the user can cause display monitor 6 to display an image including valuable notification information obtained from the outside, and also can confirm the information obtained from the outside.

In the present embodiment, for example, as shown in Figs. 6 to 8, display monitor 6 displays the associated image showing the same state as the setting state of the function shown by the operation image displayed on operation monitor 9. For example, when water supply by the HP water supply unit is in the ON state, the operation image displayed on operation monitor 9 is illuminated while the associated image displayed on display monitor 6 is also illuminated. Then, the associated image displayed on display monitor 6 represents that the HP water supply unit is in the ON state and that water is supplied during rotation of medical device 4. On the other hand, when water supply by the HP water supply unit is in the OFF state, the operation image displayed on operation monitor 9 is unilluminated while the associated image displayed on display monitor 6 is also unilluminated. Then, the associated image displayed on display monitor 6 represents that the HP water supply unit is in the OFF state, and that water is not supplied during rotation of medical device 4. However, display monitor 6 may display the associated image showing the state that is different from the setting state of the function shown by the operation image displayed on operation monitor 9.

For example, when water supply by the HP water supply unit is in the ON state, the operation image displayed on operation monitor 9 may be illuminated while the associated image displayed on display monitor 6 may be unilluminated. Then, the associated image displayed on display monitor 6 may represent that water is not supplied during rotation of medical device 4 when the HP water supply unit is brought into the OFF state. Specifically, when the user selects an operation image, display monitor 6 may display the associated image representing that the HP water supply unit is brought into an OFF state and water supply is stopped during rotation of medical device 4. On the other hand, when water supply by the HP water supply unit is in the OFF state, the operation image displayed on operation monitor 9 may be unilluminated while the associated image displayed on display monitor 6 may be illuminated. Then, the associated image displayed on display monitor 6 may represent that water is supplied during rotation of medical device 4 when the HP water supply unit is brought into an ON state. Specifically, when the user selects an operation image, display monitor 6 may display the associated image representing that the HP water supply unit is brought into an ON state and water is started to be supplied during rotation of medical device 4.

Alternatively, irrespective of whether water supply by the HP water supply unit is in the ON state or not, the associated image displayed on display monitor 6 may always be illuminated. Also, the associated image displayed on display monitor 6 may always represent that water is supplied during rotation of medical device 4 when the HP water supply unit is brought into an ON state.

In the present embodiment, an operation panel provided in the medical care apparatus installed in a hospital and the like has been exemplified. However, without being limited to such a built-in medical care apparatus, the present invention is applicable also to an operation panel provided in a carriable and portable medical care apparatus. Also in the present embodiment, the display monitor provided in the medical care apparatus has been exemplified. However, without being limited to such a built-in display monitor, a carriable and portable display terminal can also be used as a display monitor in the present invention.

Although the embodiments of the present invention have been described as above, it should be understood that the embodiments disclosed herein are illustrative and non-restrictive in every respect. The scope of the present invention is defined by the terms of the claims, and is intended to include any modifications within the meaning and scope equivalent to the terms of the claims.

## Claims

1. A medical care apparatus (10) comprising:
a first display unit (9) configured to display an operation image for operating a medical care instrument (1, 4, 2, 7);
a second display unit (6) located differently from the first display unit (9), the second display unit (6) being greater in display area than the first display unit (9); and
a display control unit (12, 15) configured to cause the second display unit (6) to display an associated image that is different from the operation image displayed on the first display unit (9), the associated image being associated with the operation image.

2. The medical care apparatus (10) according to claim 1, wherein the associated image includes detailed information associated with the operation image displayed on the first display unit (9).

3. The medical care apparatus (10) according to claim 1 or 2, wherein the display control unit (12, 15) is configured to switch the associated image displayed on the second display unit (6) according to an operation performed by a user on the operation image displayed on the first display unit (9).

4. The medical care apparatus (10) according to any one of claims 1 to 3, wherein the display control unit (12, 15) is configured to switch the associated image displayed on the second display unit (6) in accordance with a type of a selected medical care instrument (1, 4, 2, 7).

5. The medical care apparatus (10) according to any one of claims 1 to 4, wherein the associated image includes information required when a user operates the operation image.

6. The medical care apparatus (10) according to any one of claims 1 to 5, wherein the operation image is operated by a user when the user sets a function of the medical care instrument (1, 4, 2, 7), and
the associated image includes information required when the user sets the function of the medical care instrument (1, 4, 2, 7).

7. The medical care apparatus (10) according to any one of claims 1 to 6, wherein the operation image is operated by a user when the user performs maintenance of the medical care instrument (1, 4, 2, 7), and
the associated image includes information required when the user performs maintenance of the medical care instrument (1, 4, 2, 7).

8. The medical care apparatus (10) according to claim 7, wherein the display control unit (12, 15) is configured to switch the associated image displayed on the second display unit (6) in each progress of maintenance of the medical care instrument (1, 4, 2, 7) performed by a user.

9. The medical care apparatus (10) according to any one of claims 1 to 8, wherein the operation image is operated by a user when an abnormality occurs, and
the associated image includes information for notifying the user about occurrence of the abnormality.

10. The medical care apparatus (10) according to any one of claims 1 to 9, wherein the operation image is operated by a user when the user obtains information from outside, and
the associated image includes information obtained from outside.
